# FASCICULE DE BREVET EUROPEEN

(11) **EP 0 753 341 B1**
(45) Date de publication et mention de la délivrance du brevet: **23.07.1997**
(21) Numéro de dépôt: 96401489.8
(22) Date de dépôt: 05.07.1996
(51) Int. Cl.: B01F 17/00, A61K 7/40, A61K 7/42, A61K 7/13, A61K 9/107, C07C 327/22

(54) **Utilisation de composés hydrofluorocarbonés à fonction thioester dans des émulsions, applications cosmétiques ou dermatologiques**
Verwendung von Fluorokohlwasserstoffthiolestern in Emulsionen, kosmetische oder dermatologische Anwendungen
Use of hydrofluorocarbon thiolesters in emulsions, cosmetic or dermatalogical applications

(30) Priorité: 10.07.1995 FR 9508312
(43) Date de publication de la demande: 15.01.1997
(73) Titulaire: L'OREAL, 75008 Paris (FR)
(72) Inventeur: Philippe, Michel, 91320 Wissous (FR); Henrion, Jean-Christophe, 93500 Pantin (FR); Nicolas-Morgantini, Luc, 60810 Rully (FR); Dardenne, Agnès, 93600 Aulnay sous Bois (FR)
(74) Mandataire: Miszputen, Laurent

(56) Documents cités:
- EP-A- 0 195 323
- FR-A- 2 256 281
- FR-A- 2 269 523
- FR-A- 2 679 150
- FR-A- 2 684 668
- GB-A- 1 211 034
- JOURNAL OF ORGANIC CHEMISTRY, vol. 42, no. 16, 5 Août 1977, WASHINGTON, DC, US, pages 2680-2683, XP002015953 C.S. RONDESTVEDT, JR., ET AL.: "Nucleophilic displacements on beta-(perfluoroalkyl)ethyl iodides. Synthesis of acrylates containing heteroatoms"

## Description

La présente invention se rapporte à l'utilisation de composés hydrofluorocarbonés à fonction thioester dans des émulsions, ainsi qu'une composition comprenant ladite émulsion, notamment une composition cosmétique, hygiénique ou pharmaceutique. L'invention concerne également de nouveaux composés hydrofluorocarbonés à fonction thioester et leur procédé de préparation.

Il est connu d'utiliser des perfluoropolyéthers notamment dans le domaine du nettoyage, de la protection et du maquillage de la peau ou des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation en cosmétique. Certains de ses composés, les perfluorométhylisopropyléthers, sont connus sous le nom de 'Fomblin HC', et sont commercialisés par MONTEFLUO.
Il est connu, par le document FR-A-2 684 668, des composés hydrofluorocarbonés (éthers et thioéthers) présentant une bonne solubilité notamment dans les solvants classiques. Ils permettent l'obtention d'émulsions stables et homogènes.
La demanderesse s'est posée le problème d'améliorer encore la qualité et la stabilité des émulsions, et ce quelque soit la nature des huiles éventuellement employées.

L'invention a pour objet l'utilisation d'au moins un composé de formule (I) :

R_{F} - C₂H₄ - S - CO - R_{H}

dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone, comme agent stabilisant d'une émulsion.

Un autre objet de l'invention est une émulsion comprenant une phase grasse et une phase aqueuse, caractérisée en ce que la phase grasse comprend au moins un composé hydrofluorocarboné à fonction thioester, de formule (II) telle que définie ci-dessous.

R_{F}-C₂H₄-S-CO-R_{H} (II)

dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone, à l'exception des groupements -CH=CH₂ et

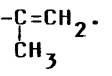

Un autre objet de l'invention sont les émulsions telles que définies dans les revendications 9 et 11.

L'invention a également pour objet une composition cosmétique, hygiénique ou dermatologique, comprenant une telle émulsion.

On a donc constaté que la présence d'au moins un composé de formule (I), ou d'un mélange de ces composés, dans une émulsion, notamment dans la phase grasse de l'émulsion, permettait d'améliorer la stabilité et la qualité de ladite émulsion, quelque soit la nature chimique de la phase grasse employée. De plus, les émulsions comprenant ledit agent stabilisant sont directement formulables, c'est-à-dire qu'elles peuvent être directement employées en particulier dans le cadre d'une application en cosmétique, en hygiène ou en pharmacie. On a également constaté que, de manière surprenante, les composés de formule (I), seuls ou en mélange, pouvaient être directement formulés en tant que constituants essentiels, voire uniques, de la phase grasse d'une émulsion, l'émulsion résultante présentant une stabilité thermodynamique et une qualité adéquates avec une utilisation dans les domaines considérés. Les émulsions comprenant les composés de formule (I) sont directement formulables, c'est-à-dire peuvent être directement employées dans les domaines hygiénique, cosmétique ou pharmaceutique.

Les composés utilisés selon l'invention sont donc de formule R_{F} - C₂H₄ - S - CO - R_{H}.

Le radical R_{F} peut de préférence représenter un groupement alkyle perfluoré, linéaire ou ramifié, ayant 4 à 10 atomes de carbone, et/ou le radical R_{H} un groupement alkyle, linéaire ou ramifié, ayant 1 à 15 atomes de carbone.

Parmi les composés de formules (I) selon l'invention, on peut citer de manière préférentielle le thiooctanoate et le thiohexanoate de 2-F-octyl éthyle, le 2-éthyl-thiohexanoate de 2'-F-hexyl éthyle et le thiohexanoate de 2-F-hexyl éthyle.

Dans la désignation des composés de formules (I), pour les groupes R_{F} perfluorés, on entendra par "F-alkyle" un groupement alkyle perfluoré. Cette notation est couramment utilisée dans la littérature pour décrire les composés perfluorés, notamment dans les articles A: Ayari, Bull Soc Chim Fr (1992), 129, p 315-318 ; A. Sismondi, Journal of Fluorine Chemistry, 59 (1992), p 127-132.

Les composés de formule II peuvent être utilisés dans et pour la préparation d'émulsions, ce qui constitue un autre objet de l'invention.

Dans un premier mode d'utilisation des composés selon l'invention, les émulsions conformes à l'invention sont constituées essentiellement d'un ou plusieurs composés de formule (II).
Par 'constitué essentiellement', on entend dans la présente description que la phase grasse comprend le/les composés de formule (II) en tant qu'huile liquide unique, lesdits composés représentant de préférence au moins 50% en poids de ladite phase grasse; ladite phase grasse peut alors comprendre 0-50% d'additifs conventionnels lipophiles autres que les huiles. De préférence, le composé de formule (II), ou le mélange de ces composés représente au moins 75% en poids de la phase grasse.

Dans un second mode d'utilisation des composés de formule (I), les émulsions selon l'invention comprennent également une ou plusieurs autres huiles usuellement utilisées dans les domaines considérés, qui peut être choisie notamment parmi les huiles hydrocarbonées, éventuellement fluorées, les huiles siliconées et les huiles perfluorées, seules ou en mélange.

La phase grasse peut de plus comprendre des additifs lipophiles classiquement utilisés dans le domaine d'application.
Parmi ces additifs, on peut citer les vitamines, les parfums, les acides gras et les lipides cireux, notamment les céramides.
Dans le cadre de ce second mode de réalisation, les composés de formule (I) peuvent en particulier être utilisés en tant qu'agent stabilisant de l'émulsion.
Ils peuvent être présents dans l'émulsion, de préférence dans la phase grasse, à raison de 0,1 à 50% en poids, de préférence 0,5-20% en poids.

La phase aqueuse des émulsions selon l'invention peut comprendre, outre l'eau, des additifs hydrophiles classiquement utilisés, tels que le glycérol et l'urée.
Le rapport phase aqueuse/phase huileuse dans l'émulsion peut être compris entre 9 et 0,1, de préférence entre 5 et 1,5.
Les émulsions peuvent ainsi se présenter sous forme eau-dans-huile ou huile-dans-eau, voire sous la forme d'une émulsion multiple.

Les émulsions conformes à l'invention peuvent être utilisées telles quelles en tant que composition, ou entrer dans le cadre de la préparation d'une composition, notamment dans les domaines cosmétique, hygiénique et dermatologique.

Ainsi, la présente invention se rapporte également à une composition cosmétique, hygiénique ou dermatologique, comprenant une émulsion telle que définie ci-dessus.
Les compositions selon l'invention peuvent se présenter en particulier sous forme d'un gel, d'un lait ou d'une crème.
En particulier, elles peuvent se présenter sous la forme d'un produit destiné au maquillage et/ou au soin de la peau ou de matières kératiniques, ou encore sous la forme d'un produit de coloration du cheveu ou d'un produit de protection solaire de la peau et/ou des matières kératiniques.

Elles peuvent comprendre en outre tout additif usuellement utilisé dans le domaine considéré, tel que des tensioactifs, des agents hydratants, des solvants organiques, des silicones, des épaississants, des adoucissants, des filtres solaires, des agents de traitement, des agents anti-mousse, des parfums, des conservateurs, des antioxydants, des séquestrants, des agents de sapidité, des agents alcalinisants ou acidifiants, des charges et des pigments, des émulsionnants, des co-émulsionnants.

Parmi les composés de formule (I) telle que définie ci-dessus, un certain nombre de produits sont connus en tant que tels, notamment ceux décrits dans FR-A-2 269 523 et GB-A-1 211 034.

GB-A-1211034 décrit la polymérisation en émulsion de certains monomères de formule (I), dans lesquels R_{H} est un groupement -CH=CH₂ on 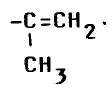 Cependant, un certain nombre de composés sont nouveaux et constituent un autre objet de l'invention. Il s'agit des composés de formule (III) suivante :

R_{F} - C₂H₄ - S - CO - R_{H} (III)

dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé, ayant de 1 à 29 atomes de carbone.

Les composés de formule (III) peuvent être préparés selon un procédé d'estérification qui consiste à réagir un thiol de formule R_{F} - C₂H₄ - SH avec un dérivé activé d'acide de formule R_{H} - CO - Y dans lequel Y représente un groupement activant, notamment de type halogène, résidu anhydride mixte ou symétrique, ou imidazole. Ce procédé constitue également un autre objet de la présente invention.

Les exemples qui suivent illustrent l'invention. Les exemples 1 à 3 décrivent un procédé de préparation de composés utilisés dans le cadre de la présente invention.

### Exemple 1 : Préparation du thiooctanoate de 2-F-octyl éthyle

Dans un réacteur de 1 litre, on introduit 240 g de 2-F-octyl-éthanethiol et 79 g de pyridine dans 500 ml d'éther diisopropylique.
On refroidit la solution à 5-8°C, tout en maintenant une certaine agitation.
On ajoute goutte-à-goutte une solution de 81,33 g de chlorure d'acide octanoïque dans 200 ml d'éther diisopropylique.
On poursuit l'agitation pendant 1 heure à 5°C, puis 4 heures supplémentaires à température ambiante.
Le mélange réactionnel est hydrolysé par ajout de 1,5 l d'eau et acidifié par HCl jusqu'à pH = 2.

La phase éthérée est recueillie, lavée à l'eau jusqu'à neutralité, puis séchée. Le solvant est évaporé et le résidu distillé.
On obtient 193 g du composé recherché, qui fige en refroidissant (rendement 64%).

L'analyse chimique du produit obtenu donne :
- Température de fusion : 31,9°C
- Température d'ébullition : 103,5/104°C à 0,3 mbar
- Spectre de RMN ¹H : conforme à la structure attendue
- Analyse élémentaire :

| | %C | % H | %F | %O | %S |
|---|---|---|---|---|---|
| Théorique | 35,65 | 3,16 | 53,26 | 2,64 | 5,29 |
| Expérimental | 35,67 | 3,12 | 52,89 | | 4,95 |

### Exemple 2 : Préparation du 2-éthyl thiohexanoate de 2'-F-hexyl éthyle

Dans un réacteur de 1 litre, on introduit 114 g de 2-F-hexyl-éthanethiol et 23,7 g de pyridine dans 200 ml d'éther diisopropylique.
On refroidit la solution à 5-8°C, tout en maintenant une certaine agitation.
On ajoute goutte-à-goutte une solution de 48,75 g de chlorure d'acide 2-éthylhexanoïque dans 100 ml d'éther diisopropylique.
On poursuit l'agitation pendant 1 heure à 5°C, puis 4 heures supplémentaires à température ambiante.
Le mélange réactionnel est hydrolysé par ajout de 1 l d'eau et acidifié par HCl jusqu'à pH = 2.
La phase éthérée est recueillie, lavée à l'eau jusqu'à neutralité, puis séchée. Le solvant est évaporé et le résidu distillé.
On obtient 100 g d'une huile incolore (rendement 66%).

L'analyse chimique du produit obtenu donne :
- Température d'ébullition : 100°C à 0,6 mbar
- Spectre de RMN ¹³C : conforme à la structure attendue
- Spectre de masse : conforme à la structure attendue
- Analyse élémentaire :

| | %C | % H | %F | %O | %S |
|---|---|---|---|---|---|
| Théorique | 37,95 | 3,78 | 48,77 | 3,16 | 6,33 |
| Expérimental | 37,61 | 3,78 | 48,52 | -- | 6,76 |

### Exemple 3 : Préparation du thiohexanoate de 2-F-hexyl éthyle

Dans un ballon de 500 ml, on introduit 76 g de 2-F-hexyl-éthanethiol et 32 g de pyridine dans 180 ml d'éther diisopropylique.
On refroidit la solution à 5-8°C, tout en maintenant une certaine agitation.
On ajoute goutte-à-goutte une solution de 27 g de chlorure d'acide hexanoïque dans 100 ml d'éther diisopropylique.
On poursuit l'agitation pendant 1 heure à 5°C, puis 4 heures supplémentaires à température ambiante.
Le mélange réactionnel est hydrolysé par ajout de 250 ml d'eau et acidifié par HCl jusqu'à pH = 2.
La phase éthérée est recueillie, lavée à l'eau jusqu'à neutralité, puis séchée.
Le solvant est évaporé et le résidu distillé.
On obtient 74 g d'une huile incolore (rendement 77%).

L'analyse chimique du produit obtenu donne :
- Température d'ébullition : 62,5/63°C à 0,25 mbar
- Spectre de masse : conforme à la structure attendue
- Spectre de RMN ¹H : conforme à la structure attendue
- Analyse élémentaire :

| | %C | % H | %F | %O | %S |
|---|---|---|---|---|---|
| Théorique | 35,16 | 3,16 | 51,63 | 3,34 | 6,70 |
| Expérimental | 35,54 | 3,22 | 51,46 | -- | 6,77 |

### Exemple 4: exemple d'émulsion

On prépare une émulsion comprenant (% en poids):

| | |
|---|---|
| . composé de l'exemple 1 | 50 % |
| . émulsionnant (polymère constitué de 80% d'acide méthacrylique et de 20% de méthacrylate de lauryle neutralisé à 80%) | 1 % |
| . eau | qsp 100 % |

L'émulsion obtenue est fluide, opaque et présente une grande homogénéité.

### Exemple 5 : exemple d'émulsion

On prépare une émulsion comprenant (% en poids):

| | |
|---|---|
| . composé de l'exemple 1 | 10 % |
| . triglycérides d'acides caprylique et caprique | 40 % |
| . émulsionnant (polymère 80% acide méthacrylique / 20% méthacrylate de lauryle, neutralisé à 80%) | 1 % |
| . eau | qsp 100 % |

L'émulsion obtenue est fluide, opaque et présente une grande homogénéité.

### Exemple 6 : exemple comparatif

Cet exemple consiste à comparer la stabilité d'une émulsion selon l'invention (exemple 4) avec une émulsion de l'art antérieur (FR 2 684 668) ayant la composition suivante (% en poids) :

| | |
|---|---|
| . 1-(2'-F-hexyléthylthio) 3-(2''-éthylhexyloxy)-2-propanol | 50 % |
| . émulsionnant (polymère 80% acide méthacrylique / 20% méthacrylate de lauryle neutralisé à 80%) | 1 % |
| . eau | qsp 100 % |

La stabilité de l'émulsion selon l'invention est nettement supérieure comparée à celle de l'émulsion de l'état de la technique.
En effet, l'émulsion selon l'invention reste stable pendant plus de 70 jours, lors d'une conservation à 20°C, alors que l'émulsion selon l'état de la technique ne reste stable qu'environ 15 jours; on observe ensuite une démixtion de l'émulsion.

### Exemple 7 : exemple comparatif

On compare la stabilité d'émulsions comprenant un composé selon l'invention, avec les mêmes émulsions sans composé.
Dans tous ces exemples, l'émulsionnant est un polymère 80% acide méthacrylique / 20% méthacrylate de lauryle, neutralisé à 80%.

On obtient les résultats suivants :

### 1ère comparaison

| *Invention (% en poids)* | |
|---|---|
| . composé de l'exemple 1 | 10 % |
| . triglycérides d'acides caprylique et caprique | 40 % |
| . émulsionnant | 1 % |
| . eau | qsp 100 % |

| *Art antérieur* | |
|---|---|
| . triglycérides d'acides caprylique et caprique | 50 % |
| . émulsionnant | 1 % |
| . eau | qsp 100 % |

La stabilité de l'émulsion selon l'invention est nettement supérieure comparée à celle de l'émulsion de l'état de la technique. L'émulsion selon l'invention reste stable pendant plus de 70 jours, lors d'une conservation à 20°C, alors que l'émulsion selon l'état de la technique ne reste stable qu'environ 10 jours dans les mêmes conditions.

### 2ème comparaison

| *Invention (% en poids)* | |
|---|---|
| . composé de l'exemple 2 | 10 % |
| . palmitate d'éthyl-2-hexyle | 40 % |
| . émulsionnant | 1 % |
| . eau | qsp 100 % |

| *Art antérieur* | |
|---|---|
| . palmitate d'éthyl-2-hexyle | 50 % |
| . émulsionnant | 1 % |
| . eau | qsp 100 % |

La stabilité de l'émulsion selon l'invention est nettement supérieure comparée à celle de l'émulsion de l'état de la technique. L'émulsion selon l'invention reste stable pendant plus de 70 jours, lors d'une conservation à 20°C, alors que l'émulsion selon l'état de la technique ne reste stable qu'environ 4 ou 5 jours dans les mêmes conditions.

### 3ème comparaison

| *Invention (% en poids)* | |
|---|---|
| . composé de l'exemple 1 | 10 % |
| . cyclopentadiméthylsiloxane | 40 % |
| . émulsionnant | 1 % |
| . eau | qsp 100 % |

| *Art antérieur* | |
|---|---|
| . cyclopentadiméthylsiloxane | 50 % |
| . émulsionnant | 1 % |
| . eau | qsp 100 % |

La stabilité de l'émulsion selon l'invention est nettement supérieure comparée à celle de l'émulsion de l'état de la technique.
L'émulsion selon l'invention reste stable pendant plus de 70 jours, lors d'une conservation à 20°C, alors que l'émulsion selon l'état de la technique ne reste stable qu'environ 8-9 jours dans les mêmes conditions.

### 4ème comparaison

| *Invention (% en poids)* | |
|---|---|
| . composé de l'exemple 3 | 10 % |
| . huile de vaseline | 40 % |
| . émulsionnant | 1 % |
| . eau | qsp 100 % |

| *Art antérieur* | |
|---|---|
| . huile de vaseline | 50 % |
| . émulsionnant | 1 % |
| . eau | qsp 100 % |

La stabilité de l'émulsion selon l'invention est nettement supérieure comparée à celle de l'émulsion de l'état de la technique.
L'émulsion selon l'invention reste stable pendant plus de 70 jours, lors d'une conservation à 20°C, alors que l'émulsion selon l'état de la technique ne reste stable qu'à peine une journée dans les mêmes conditions.

## Revendications

1. Utilisation d'au moins un composé de formule (I)
R_{F} - C₂H₄ - S - CO - R_{H} (I)
dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone,
comme agent stabilisant, dans une émulsion.

2. Utilisation selon la revendication 5, dans laquelle l'agent stabilisant est présent dans l'émulsion en une quantité de 0,1 à 50% en poids, de préférence 0,5-20% en poids.

3. Utilisation d'au moins un composé de formule (II)
R_{F} - C₂H₄ - S - CO - R_{H} (II)
dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone, à l'exception des groupements -CH=CH₂ et 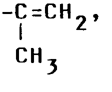 dans et pour la préparation d'une émulsion.

4. Utilisation selon la revendication 1, caractérisée par le fait que R_{F} représente un groupement alkyle perfluoré ayant de 4 à 10 atomes de carbone et/ou R_{H} représente un groupement alkyle ayant de 1 à 15 atomes de carbone.

5. Utilisation selon l'une des revendications précédentes, caractérisée par le fait que le composé de formule (I) est choisi dans le groupe constitué par le thiooctanoate de 2-F-octyl éthyle, le thiohexanoate de 2-F-octyl éthyle, le 2-éthyl-thiohexanoate de 2'-F-hexyl éthyle et le thiohexanoate de 2-F-hexyl éthyle.

6. Emulsion comprenant une phase grasse et une phase aqueuse, caractérisée en ce que la phase grasse comprend au moins un composé hydrofluorocarboné à fonction thioester, de formule (II):
R_{F} - C₂H₄ - S - CO - R_{H} (II)
dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone, à l'exception des groupements -CH=CH₂ et

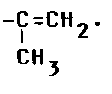

7. Emulsion selon la revendication 6, dans laquelle la phase grasse est essentiellement constituée de composés de formule (II).

8. Emulsion selon l'une des revendications 6 ou 7, dans laquelle la phase grasse comprend au moins 50% en poids de composés de formule (II), de préférence au moins 75% en poids.

9. Emulsion comprenant une phase grasse et une phase aqueuse, caractérisée en ce que la phase grasse comprend au moins un composé hydrofluorocarboné à fonction thioester, de formule (I) :
R_{F} - C₂H₄ - S - CO - R_{H} (I)
dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 à 29 atomes de carbone, et dans laquelle la phase grasse comprend en outre au moins une huile, qui peut être choisie parmi les huiles hydrocarbonées, éventuellement fluorées, les huiles siliconées, les huiles perfluorées, seules ou en mélange.

10. Emulsion selon la revendication 9, dans laquelle la phase grasse comprend 0,1 à 50% en poids, de préférence 0,5-20% en poids, de composés de formule (I).

11. Emulsion comprenant une phase grasse et une phase aqueuse, caractérisée en ce que la phase grasse comprend au moins un composé hydrofluorocarboné a fonction thioester, de formule (I) :
R_{F} - C₂H₄ - S - CO - R_{H} (I)
dans laquelle :
R_{F} représente un groupement alkyle, linéaire ou ramifié, perfluoré ayant de 4 à 20 atomes de carbone, et
R_{H} représente un groupement alkyle, linéaire ou ramifié, saturé ou insaturé, ayant de 1 a 29 atomes de carbonne, et dans laquelle la phase grasse comprend 0,1 a 50% en poids, de préférence 0,5-20% en poids, de composés de formule (I).

12. Emulsion selon l'une des revendications 6 à 11, se présentant sous forme eau-dans-huile ou huile-dans-eau, ou sous la forme d'une émulsion multiple.

13. Composition cosmétique, hygiénique ou dermatologique, caractérisée en ce qu'elle comprend une émulsion selon l'une des revendications 6 à 12.

14. Composition selon la revendication 13, se présentant sous la forme d'un produit destiné au maquillage et/ou au soin de la peau ou de matières kératiniques, ou encore sous la forme d'un produit de coloration du cheveu ou d'un produit de protection solaire de la peau et/ou des matières kératiniques.

15. Composé hydrofluorocarboné à fonction thioester, de formule (III):
R_{F} - C₂H₄ - S - CO - R_{H} (III)
dans laquelle :
R_{F} représente un groupement alkyle, linéaire ou ramifié, perfluoré ayant de 4 a 20 atomes de carbone, et
R_{H} représente un groupement alkyle, linéaire ou ramifié, saturé, ayant de 1 à 29 atomes de carbone.

16. Composé selon la revendication 15, dans lequel R_{F} représente un groupement alkyle perfluoré ayant de 4 à 10 atomes de carbone et/ou R_{H} représente un groupement alkyle ayant de 1 à 15 atomes de carbone.

17. Composé selon l'une des revendications 15 ou 16, choisi dans le groupe constitué par le thiooctanoate de 2-F-octyl éthyle, le thiohexanoate de 2-F-octyl éthyle, le 2-éthyl-thiohexanoate de 2'-F-hexyl éthyle et le thiohexanoate de 2-F-hexyl éthyle.

18. Procédé de préparation des composés de formule (III) consistant à faire réagir un thiol de formule R_{F} - C₂H₄ - SH avec un dérivé activé d'acide de formule R_{H} - CO - Y dans lequel Y représente un groupement activant, notamment de type halogène, résidu anhydride mixte ou symétrique, ou imidazole.

## Claims

1. Use of at least one compound of formula (I)
R_{F}-C₂H₄-S-CO-R_{H} (I)
in which:
R_{F} represents a linear or branched perfluoroalkyl group having from 4 to 20 carbon atoms, and
R_{H} represents a linear or branched, saturated or unsaturated alkyl group having from 1 to 29 carbon atoms, as a stabilizing agent in an emulsion.

2. Use according to Claim 1, in which the stabilizing agent is present in the emulsion in an amount of from 0.1 to 50 % by weight, preferably 0.5-20 % by weight.

3. Use of at least one compound of formula (II)
R_{F}-C₂H₄-S-CO-R_{H} (II)
in which:
R_{F} represents a linear or branched perfluoroalkyl group having from 4 to 20 carbon atoms, and
R_{H} represents a linear or branched, saturated or unsaturated alkyl group having from 1 to 29 carbon atoms, with the exception of -CH=CH₂ and 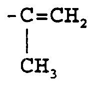 groups,
in and for the preparation of an emulsion.

4. Use according to Claim 1, characterized in that R_{F} represents a perfluoroalkyl group having from 4 to 10 carbon atoms and/or R_{H} represents an alkyl group having from 1 to 15 carbon atoms.

5. Use according to one of the preceding claims, characterized in that the compound of formula (I) is chosen from the group consisting of 2-F-octylethyl thiooctanoate, 2-F-octylethyl thiohexanoate, 2'-F-hexylethyl 2-ethylthiohexanoate and 2-F-hexylethyl thiohexanoate.

6. Emulsion comprising a fatty phase and an aqueous phase, characterized in that the fatty phase comprises at least one fluorohydrocarbon compound containing a thioester function, of formula (II)
R_{F}-C₂H₄-S-CO-R_{H} (II)
in which:
R_{F} represents a linear or branched perfluoroalkyl group having from 4 to 20 carbon atoms, and
R_{H} represents a linear or branched, saturated or unsaturated alkyl group having from 1 to 29 carbon atoms, with the exception of -CH=CH₂ and 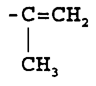 groups.

7. Emulsion according to Claim 6, in which the fatty phase consists essentially of compounds of formula (II).

8. Emulsion according to either of Claims 6 and 7, in which the fatty phase comprises at least 50 % by weight of compounds of formula (II), preferably at least 75 % by weight.

9. Emulsion comprising a fatty phase and an aqueous phase, characterized in that the fatty phase comprises at least one fluorohydrocarbon compound containing a thioester function, of formula (I)
R_{F}-C₂H₄-S-CO-R_{H} (I)
in which:
R_{F} represents a linear or branched perfluoroalkyl group having from 4 to 20 carbon atoms, and
R_{H} represents a linear or branched, saturated or unsaturated alkyl group having from 1 to 29 carbon atoms, and in which the fatty phase also comprises at least one oil which may be chosen from optionally fluorinated hydrocarbon oils, silicone oils and perfluoro oils, alone or as a mixture.

10. Emulsion according to Claim 9, in which the fatty phase comprises 0.1 to 50 % by weight, preferably 0.5-20 % by weight, of compounds of formula (I).

11. Emulsion comprising a fatty phase and an aqueous phase, characterized in that the fatty phase comprises at least one fluorohydrocarbon compound containing a thioester function, of formula (I)
R_{F}-C₂H₄-S-CO-R_{H} (I)
in which:
R_{F} represents a linear or branched perfluoroalkyl group having from 4 to 20 carbon atoms, and
R_{H} represents a linear or branched, saturated or unsaturated alkyl group having from 1 to 29 carbon atoms, and in which the fatty phase comprises 0.1 to 50 % by weight, preferably 0.5-20 % by weight, of compounds of formula (I).

12. Emulsion according to one of Claims 6 to 11, which is in water-in-oil or oil-in-water form or is in the form of a multiple emulsion.

13. Cosmetic, hygiene or dermatological composition, characterized in that it comprises an emulsion according to one of Claims 6 to 12.

14. Composition according to Claim 13, in the form of a product intended for making up the skin and/or for skin care or for the care of keratin substances, or alternatively in the form of a hair dyeing product or a product for protecting the skin and/or keratin substances against sunlight.

15. Fluorohydrocarbon compound containing a thioester function, of formula (III)
R_{F}-C₂H₄-S-CO-R_{H} (III)
in which:
R_{F} represents a linear or branched perfluoroalkyl group having from 4 to 20 carbon atoms, and
R_{H} represents a linear or branched, saturated alkyl group having from 1 to 29 carbon atoms.

16. Compound according to Claim 15, in which R_{F} represents a perfluoroalkyl group having from 4 to 10 carbon atoms and/or R_{H} represents an alkyl group having from 1 to 15 carbon atoms.

17. Compound according to either of Claims 15 and 16, chosen from the group consisting of 2-F-octylethyl thiooctanoate, 2-F-octylethyl thiohexanoate, 2'-F-hexylethyl 2-ethylthiohexanoate and 2-F-hexylethyl thiohexanoate.

18. Process for the preparation of the compounds of formula (III), which consists in reacting a thiol of formula R_{F}-C₂H₄-SH with an acid-activated derivative of formula R_{H}-CO-Y in which Y represents an activating group, in particular one of halogen, mixed or symmetrical anhydride residue or imidazole type.

## Patentansprüche

1. Verwendung einer oder mehrerer Verbindungen der Formel (I):
R_{F}-C₂H₄-S-CO-R_{H} (I),
worin bedeuten:
R_{F} eine geradkettige oder verzweigte perfluorierte Alkylgruppe mit 4 bis 20 Kohlenstoffatomen und
R_{H} eine geradkettige oder verzweigte gesättigte oder ungesättigte Alkylgruppe mit 1 bis 29 Kohlenstoffatomen,
als Stabilisierungsmittel in einer Emulsion.

2. Verwendung nach Anspruch 1, wobei das Stabilisierungsmittel in der Emulsion in einem Mengenanteil von 0,1 bis 50 Gew.-% und vorzugsweise von 0,5 bis 20 Gew.-% vorliegt.

3. Verwendung einer oder mehrerer Verbindungen der Formel (II):
R_{F}-C₂H₄-S-CO-R_{H} (II),
worin bedeuten:
R_{F} eine geradkettige oder verzweigte perfluorierte Alkylgruppe mit 4 bis 20 Kohlenstoffatomen und
R_{H} eine geradkettige oder verzweigte gesättigte oder ungesättigte Alkylgruppe mit 1 bis 29 Kohlenstoffatomen außer den Gruppen -CH=CH₂ und 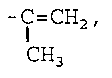 zur Herstellung einer Emulsion.

4. Verwendung nach Anspruch 1, wobei R_{F} eine perfluorierte Alkylgruppe mit 4 bis 10 Kohlenstoffatomen und/oder R_{H} eine Alkylgruppe mit 1 bis 15 Kohlenstoffatomen bedeutet.

5. Verwendung nach einem der vorhergehenden Ansprüche, dadurch gekennzeichnet, daß die Verbindung der Formel (I) ausgewählt ist unter: Thiooctansäure-2-F-octyl-ethylester, Thiohexansäure-2-F-octyl-ethylester, 2-Ethylthiohexansäure-2'-F-hexyl-ethylester und Thiohexansäure-2-F-hexyl-ethylester

6. Emulsion, die eine Fettphase und eine wäßrige Phase enthält, dadurch gekennzeichnet, daß die Fettphase eine oder mehrere Fluorkohlenwasserstoffverbindungen mit Thioestergruppe der Formel (II):
R_{F}-C₂H₄-S-CO-R_{H} (II),
enthält, worin bedeuten:
R_{F} eine geradkettige oder verzweigte perfluorierte Alkylgruppe mit 4 bis 20 Kohlenstoffatomen und
R_{H} eine geradkettige oder verzweigte gesättigte oder ungesättigte Alkylgruppe mit 1 bis 29 Kohlenstoffatomen außer den Gruppen -CH=CH₂ und

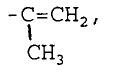

7. Emulsion nach Anspruch 6, wobei die Fettphase im wesentlichen aus Verbindungen der Formel (II) besteht.

8. Emulsion nach Anspruch 6 oder 7, wobei die Fettphase mindestens 50 Gew.-% Verbindungen der Formel (II) und vorzugsweise mindestens 75 Gew.-% enthält.

9. Emulsion, die eine Fettphase und eine wäßrige Phase enthält, dadurch gekennzeichnet, daß die Fettphase eine oder mehrere Fluorkohlenwasserstoffverbindungen mit Thioestergruppe der Formel (I):
R_{F}-C₂H₄-S-CO-R_{H} (I),
enthält, worin bedeuten:
R_{F} eine geradkettige oder verzweigte perfluorierte Alkylgruppe mit 4 bis 20 Kohlenstoffatomen und
R_{H} eine geradkettige oder verzweigte gesättigte oder ungesättigte Alkylgruppe mit 1 bis 29 Kohlenstoffatomen,
wobei die Fettphase mindestens ein Öl enthält, das unter Kohlenwasserstoffölen, die gegebenenfalls fluoriert sind, Siliconölen und perfluorierten Ölen sowie entsprechenden Gemischen ausgewählt ist.

10. Emulsion nach Anspruch 9, wobei die Fettphase 0,1 bis 50 Gew.-% Verbindungen der Formel (I) und vorzugsweise 0,5 bis 20 Gew.-% enthält.

11. Emulsion, die eine Fettphase und eine wäßrige Phase enthält, dadurch gekennzeichnet, daß die Fettphase eine oder mehrere Fluorkohlenwasserstoffverbindungen mit Thioestergruppe der Formel (I):
R_{F}-C₂H₄-S-CO-R_{H} (I),
enthält, worin bedeuten:
R_{F} eine geradkettige oder verzweigte perfluorierte Alkylgruppe mit 4 bis 20 Kohlenstoffatomen und
R_{H} eine geradkettige oder verzweigte gesättigte oder ungesättigte Alkylgruppe mit 1 bis 29 Kohlenstoffatomen,
wobei die Fettphase 0,1 bis 50 Gew.-% Verbindungen der Formel (I) und vorzugsweise 0,5 bis 20 Gew.-% enthält.

12. Emulsion nach einem der Ansprüche 6 bis 11, die in Form einer Wasser-in-Öl-Emulsion oder Öl-in-Wasser-Emulsion oder in Form einer multiplen Emulsion vorliegt.

13. Kosmetische, hygienische oder pharmazeutische Zusammensetzung, dadurch gekennzeichnet, daß sie eine Emulsion nach einem der Ansprüche 6 bis 12 enthält.

14. Zusammensetzung nach Anspruch 13, die in Form eines Produkts zum Schminken und/oder zur Pflege der Haut oder von Keratinfasern oder in Form eines Produkts zum Färben von Haaren oder eines Produkts zum Sonnenschutz der Haut und/oder von Keratinfasern vorliegt.

15. Fluorkohlenwasserstoffverbindungen mit Thioestergruppe der Formel (III):
R_{F}-C₂H₄-S-CO-R_{H} (III),
worin bedeuten:
R_{F} eine geradkettige oder verzweigte perfluorierte Alkylgruppe mit 4 bis 20 Kohlenstoffatomen und
R_{H} eine geradkettige oder verzweigte gesättigte Alkylgruppe mit 1 bis 29 Kohlenstoffatomen.

16. Verbindungen nach Anspruch 15, wobei R_{F} eine perfluorierte Alkylgruppe mit 4 bis 10 Kohlenstoffatomen und/oder R_{H} eine Alkylgruppe mit 1 bis 15 Kohlenstoffatomen bedeutet.

17. Verbindungen nach einem der Ansprüche 15 oder 16, die ausgewählt sind unter: Thiooctansäure-2-F-octyl-ethylester, Thiohexansäure-2-F-octyl-ethylester, 2-Ethylthiohexansäure-2'-F-hexyl-ethylester und Thiohexansäure-2-F-hexyl-ethylester.

18. Verfahren zur Herstellung der Verbindungen der Formel (III), das darin besteht, ein Thiol der Formel R_{F}-C₂H₄-SH mit einem aktivierten Säurederivat der Formel R_{H}-CO-Y umzusetzen, wobei die Gruppe Y eine aktivierende Gruppe ist, insbesondere vom Typ Halogen, gemischter oder symmetrischer Anhydridrest oder Imidazol.
